# EUROPEAN PATENT APPLICATION

(11) **EP 4 129 326 A1**
(43) Date of publication of application: **08.02.2023**
(21) Application number: 21775220.3
(22) Date of filing: 12.03.2021
(51) Int. Cl.: A61K 39/00, A61P 15/00, C07K 14/28, C07K 7/06, C07K 14/705, C12N 15/63

(54) **RECOMBINANT PROTEIN FOR NEUTERING OR SPAYING ANIMAL, AND VACCINE COMPOSITION COMPRISING SAME**

(30) Priority: 24.03.2020 KR 20200035898
(71) Applicant: Bioapplications Inc., Pohang-si, Gyeongsangbuk-do 37668 (KR)
(72) Inventor: SOHN, Eun-Ju, Pohang-Si, Gyeongsangbuk-do 37668 (KR); LEE, Yong Jik, Pohang-Si, Gyeongsangbuk-do 37668 (KR)
(74) Representative: Dehns
(86) International application number: PCT/KR2021/003097
(87) International publication number: WO 2021/194142

(57) **Abstract**

The present invention relates to a vaccine composition which is form neutering or spaying an animal and comprises a recombinant protein in which cholera toxin B subunit (CTB) and gonadotropin-releasing hormone (GnRH) are fused. More specifically, provided are: a recombinant protein for neutering or spaying an animal and for inducing antibodies against GnRH; a recombinant vector for producing the recombinant protein; a vaccine composition for neutering or spaying an animal, the vaccine composition comprising the recombinant protein; and a method for neutering or spaying an animal by using the vaccine composition. The vaccine composition according to the present invention induces antibodies against GnRH in an individual, thereby atrophying the ovaries or testes thereof. Therefore, the present invention can, at low cost, a high level of safety, and with minimal side effects, replace surgical procedures for neutering and spaying, and be beneficially used to neuter or spay an animal.

## Description

### [Technical Field]

The present invention relates to a vaccine composition for neutering an animal, and more particularly, to a recombinant protein for neutering an animal to induce an antibody against gonadotropin-releasing hormone (GnRH), a recombinant vector for producing the same, a vaccine composition for neutering an animal, which includes the recombinant protein as an active ingredient, and a neutering method using the same.

This application claims priority to and the benefit of Korean Patent Application No. 10-2020-0035898, filed on March 24, 2020, the disclosure of which is incorporated in the specification and the drawings thereof are incorporated herein by reference in its entirety.

### [Background Art]

Neutering is the removal of the gonads of an animal to eliminate the reproductive function, and refers to a phenomenon in which the secretion of gonadotropin is cut off by removing the testes of a male or the ovaries of a female, and thus infertility occurs and secondary sexual characteristics do not appear or are degenerated. Neutering is being conducted for the purpose of increasing research and utility value in medicine, biology, and animal husbandry. Particularly, in the livestock industry, neutering is mainly performed on males not only to treat an animal disease, but to increase its utility value, and this is because ovarian removal is extremely difficult and is less effective. Male animals that have undergone neutering are gentle by nature and docile, enable breeding of male and female animals together due to the loss of reproduction ability, have thinner and softer muscle fibers, thereby improving the quality of meat, and increase the percentage of flesh attached to bones, which become thinner, like female animals. In addition, neutering lowers the generation of boar taint, thereby reducing male odor.

Neutering methods include surgical methods such as scrotal incision, spermatic vein ligation, testicular torsion, scrotal root ligation, testicular extrusion, partial castration, and bloodless castration (Burdizzo method), and also includes a method of applying a certain amount of radiation to radiation-sensitive gonads to lose their ability. However, physical neutering is problematic in terms of animal welfare because it causes very serious pain and stress in animals, so European countries such as Switzerland, Norway, Belgium, and the Netherlands decided to ban the physical neutering of animals around 2009, and South Korea is also considering a similar action thereto.

Therefore, recently, as a method of solving the problems of neutering surgery, neutering methods using a vaccine are being actively studied. Most of the neutering methods using a vaccine are performed using a protein capable of inducing an immune response to GnRH, and this is because GnRH secreted from the hypothalamus stimulates the secretion of the follicle-stimulating hormone-releasing hormone (FSH) and luteinizing hormone (LH) from the pituitary gland. FSH promotes follicle maturation, estrogen secretion, and spermatogenesis and LH promotes the development of reproductive organs, testosterone secretion, and ovulation. In addition, LH and FSH stimulate androstenone secretion in the testis of male animals, and androstenone accumulates in adipose tissue, generating boar taint.

As a neutering vaccine developed so far, there is Improvac (Pfizer), but it has a disadvantage in that a strong immune response is not induced. Accordingly, while studying to develop a vaccine that induces a stronger immune response than the conventional neutering vaccine, the inventors confirmed that a CTB-GnRH recombinant protein of the present invention has superior antigenicity to the conventionally known neutering vaccine, thereby exhibiting an excellent antibody-forming effect against GnRH and a resulting strong inhibitory effect on sexual maturation of an animal, and thus the present invention was completed.

### [Disclosure]

### [Technical Problem]

The present invention is provided to solve the conventional technical problems described above, a vaccine including a recombinant protein for neutering an animal fused with a carrier protein to artificially improve the antigenicity of gonadotropin-releasing hormone (GnRH) was developed, and thus the present invention was completed.

Therefore, the present invention is directed to providing a vaccine composition for neutering an animal, which comprises a recombinant protein in which cholera toxin B subunit (CTB) and n copies of gonadotropin-releasing hormone (GnRH) are fused as an active ingredient, wherein n is an integer from 1 to 20.

The present invention is also directed to providing a method of neutering an animal, which comprises administering the vaccine composition according to the present invention into an animal excluding a human.

The present invention is also directed to providing a recombinant vector for neutering an animal, which comprises GnRH gene consisting of a base sequence of SEQ ID NO: 3 and a CTB gene consisting of a base sequence of SEQ ID NO: 5.

The present invention is also directed to providing a recombinant protein for neutering an animal prepared using the recombinant vector according to the present invention.

The present invention is also directed to providing a transformant which is transformed with the recombinant vector according to the present invention.

However, technical problems to be solved in the present invention are not limited to the above-described problems, and other problems which are not described herein will be fully understood by those of ordinary skill in the art from the following descriptions.

### [Technical Solution]

To achieve the purpose of the present invention described above, the present invention provides a vaccine composition for neutering an animal, which comprises a recombinant protein in which CTB and n copies of GnRH are fused as an active ingredient, wherein n is an integer from 1 to 20.

The present invention also provides a neutering method, which comprises administering a vaccine composition including a recombinant protein in which CTB and n copies of GnRH are fused as an active ingredient, wherein n is an integer from 1 to 20, into a subject excluding a human.

The present invention also provides the use of a recombinant protein in which CTB and n copies of GnRH are fused and n is an integer from 1 to 20 for neutering.

Moreover, the present invention provides the use of a recombinant protein in which CTB and n copies of GnRH are fused and n is an integer from 1 to 20 for the manufacture of a vaccine used in neutering.

In one embodiment of the present invention, the GnRH may consist of an amino acid sequence of SEQ ID NO: 1, but the present invention is not limited thereto.

In another embodiment of the present invention, the CTB may consist of an amino acid sequence of SEQ ID NO: 4, but the present invention is not limited thereto.

In still another embodiment of the present invention, the n may be 6, but the present invention is not limited thereto.

In yet another embodiment of the present invention, when the n is 6, the GnRH may consist of an amino acid sequence of SEQ ID NO: 2, but the present invention is not limited thereto.

In yet another embodiment of the present invention, when the n is 2 or more, the GnRHs may be connected with each other using an alanine-glycine-alanine linker, but the present invention is not limited thereto.

In one embodiment of the present invention, in the recombinant protein, a porcine Fc fragment (pFc2) may be further fused.

In another embodiment of the present invention, the pFc2 may consist of an amino acid sequence of SEQ ID NO: 6, but the present invention is not limited thereto.

In still another embodiment of the present invention, the CTB may be fused in the N-terminus direction of GnRH, and the pFc2 may be fused in the C-terminus direction of GnRH, but the present invention is not limited thereto.

In one embodiment of the present invention, in the recombinant protein, a chaperone-binding protein (BiP) may be further fused.

In another embodiment of the present invention, the BiP may consist of an amino acid sequence of SEQ ID NO: 8, but the present invention is not limited thereto.

In still another embodiment of the present invention, the CTB may be fused in the N-terminus direction of GnRH, and the BiP may be fused in the N-terminus direction of CTB, but the present invention is not limited thereto.

In one embodiment of the present invention, in the recombinant protein, His-Asp-Glu-Leu (HDEL) may be further fused.

In another embodiment of the present invention, the HDEL may be encoded by a base sequence of SEQ ID NO: 11, but the present invention is not limited thereto.

In still another embodiment of the present invention, the CTB may be fused in the N-terminus direction of GnRH, and the HDEL may be fused in the C-terminus direction of GnRH, but the present invention is not limited thereto.

In addition, in one embodiment of the present invention, in the recombinant protein, BiP, CTB, GnRH, pFc2, and HDEL may be sequentially fused, but the present invention is not limited thereto.

In another embodiment of the present invention, the recombinant protein may consist of an amino acid sequence of SEQ ID NO: 12, but the present invention is not limited thereto.

In one embodiment of the present invention, the vaccine composition may further include an adjuvant.

In another embodiment of the present invention, the adjuvant may be an Emulsigen-based adjuvant, but the present invention is not limited thereto.

In addition, the present invention provides a method of neutering an animal, which includes administering the vaccine composition according to the present invention to an animal excluding a human.

In one embodiment of the present invention, the animal may be a dog, a whale, a cat, a gorilla, a goose, a guinea pig, a pheasant, a camel, a roe deer, a mule, a chicken, a donkey, a dolphin, a pig, a llama, a horse, a buffalo, a deer, a cow, a reindeer, an alpaca, a yak, sheep, a goat, an orangutan, a duck, a monkey, a ferret, a rat, a turkey, an ostrich, or a rabbit, but the present invention is not limited thereto.

In addition, the present invention provides a recombinant vector for neutering an animal, which includes a GnRH gene consisting of a base sequence of SEQ ID NO: 3 and a CTB gene consisting of a base sequence of SEQ ID NO: 5.

In one embodiment of the present invention, the recombinant vector may further include a polynucleotide encoding pFc2 consisting of an amino acid sequence of SEQ ID NO: 6, but the present invention is not limited thereto.

In another embodiment of the present invention, the recombinant vector may further include a polynucleotide encoding BiP consisting of an amino acid sequence of SEQ ID NO: 8, but the present invention is not limited thereto.

In still another embodiment of the present invention, the recombinant vector may further include a HDEL gene consisting of an amino acid sequence of SEQ ID NO: 11, but the present invention is not limited thereto.

In yet another embodiment of the present invention, the recombinant vector may include a polynucleotide encoding an amino acid sequence of SEQ ID NO: 12, or a base sequence of SEQ ID NO: 13, but the present invention is not limited thereto.

In addition, the present invention provides a recombinant protein for neutering an animal, prepared using the recombinant vector according to the present invention.

In addition, the present invention provides a transformant which is transformed with the recombinant vector according to the present invention.

### [Advantageous Effects]

The present invention relates to a vaccine composition for neutering an animal, which comprises a recombinant protein in which a cholera toxin B subunit (CTB) and gonadotropin-releasing hormone (GnRH) are fused. The recombinant protein comprising the polypeptide sequence of GnRH is used to induce an antibody against GnRH in a subject to which the vaccine composition is administered, and through this, ovarian and testicular atrophy effects were confirmed. Therefore, the present invention can be effectively used to neuter an animal at low cost and with high safety and minimal side effects.

### [Description of Drawings]

FIG. 1 shows an expression cassette for preparing a recombinant GnRH protein according to one embodiment of the present invention (BiP, chaperone binding protein; CTB, cholera toxin B subunit; pFc2, porcine Fc fragment; HDEL, histidine-aspartic acid-glutamic acid-leucine).
FIG. 2 is the result of isolating and purifying a recombinant GnRH protein according to one embodiment of the present invention.
FIG. 3 shows the schedule by which a vaccine composition containing a recombinant GnRH protein according to one embodiment of the present invention is administered into a male rat.
FIG. 4 shows the comparison in size of testes by extracting the testes after a vaccine composition containing a recombinant GnRH protein according to one embodiment of the present invention is administered three times into a male rat.
FIG. 5 shows the result of measuring the concentration of testosterone in the blood after a vaccine composition containing a recombinant GnRH protein according to one embodiment of the present invention is administered three times into a male rat, and the upper and middle diagrams show standards, and the lower diagram shows the result of measuring each group.
FIG. 6 shows the schedule by which a vaccine composition containing a recombinant GnRH protein according to one embodiment of the present invention is administered into a female rat.
FIGS. 7 and 8 show the results of an antigen-antibody reaction of serum performed to confirm whether an antibody against GnRH was produced by a neutering vaccine according to one embodiment of the present invention, wherein FIG. 7 shows the result of administering a vaccine composition containing CTB-GnRH, and FIG. 8 shows the result of administering a vaccine composition containing Fe-GnRH.
FIG. 9 shows the comparison in size of ovaries by extracting the ovaries after a vaccine composition containing a recombinant GnRH protein according to one embodiment of the present invention is administered three times into a female rat.

### [Modes of the Invention]

The inventors analyzed changes in the size and weight of testes or ovaries and a change in the concentration of a sex hormone in the blood after the inoculation of a recombinant gonadotropin-releasing hormone (GnRH) protein into 4-week-old male and female rats. As a result, by confirming that the group vaccinated with the recombinant GnRH protein of the present invention had a reduction in the size and weight of testes or ovaries and a decreased concentration of blood sex hormone compared to a control, it was confirmed that the vaccine composition including the recombinant GnRH protein according to the present invention had a neutering effect. Thus, the present invention was completed.

Therefore, the present invention may provide a vaccine composition for neutering an animal, which comprises a recombinant protein in which a cholera toxin B subunit (CTB) and n copies of gonadotropin-releasing hormone (GnRH) are fused as an active ingredient, wherein n is an integer from 1 to 20.

The term "cholera toxin B subunit (CTB)" used herein refers to a polypeptide having binding activity to a host cell, and is preferably encoded by a polynucleotide including a base sequence of SEQ ID NO: 5, and most preferably the polynucleotide represented by SEQ ID NO: 5. However, the polynucleotide is encoded by a base sequence having 80% or more, more preferably 90% or more, and even more preferably 95% or more sequence homology with the base sequence of SEQ ID NO: 5. For example, the polynucleotide has, for example, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence homology with the base sequence of SEQ ID NO: 5. The "% sequence homology" with a polynucleotide is identified by comparing two optimally-arranged sequences and a comparative region, and a part of the polynucleotide sequence in the comparative region may have an addition or deletion (i.e., gap) compared to a reference sequence for the optimal arrangement of two sequences (not including addition or deletion). Cholera toxin is a complex of A1 and A2 subunits and five B subunits, and CTB binds to microfold cells (M cells) of the mucosal immune system for internalization and activation of the cholera toxin. Here, the CTB allows to an A subunit or an antigenic protein binding to the CTB to pass through a cell membrane after specifically binding to the GM1-ganglioside of the cell membrane bilayer. The CTB may be used as a potent mucosal adjuvant and carrier molecule for reinforcing mucosal immunity by improving the immunogenicity of an antigen through an increase in absorption of the bound antigen passing through the mucosal barrier and efficient presentation of the bound antigen.

The term "polynucleotide" used herein refers to an oligomer or polymer including two or more nucleotides or derivatives thereof generally connected by a phosphate ester bond, including deoxyribonucleic acid (DNA) and ribonucleic acid (RNA). The polynucleotide also includes, for example, nucleotide analogs, and DNA and RNA derivatives having "backbone" bonds excluding a phosphodiester bond, such as a phosphotriester bond, a phosphoramidate bond, a phosphorothioate bond, a thioester bond, or a peptide bond (peptide nucleic acid). The polynucleotides include single-stranded and/or double-stranded polynucleotides, such as deoxyribonucleic acid (DNA) and ribonucleic acid (RNA) as well as analogs of any one of RNA and DNA.

The term "GnRH" used herein refers to gonadotropin-releasing hormone, and is preferably GnRH consisting of the amino acid sequence of SEQ ID NO: 1, but any one that can induce an immune response to GnRH can be used without limitation. In addition, as the GnRH of the present invention, a variant of SEQ ID NO: 1 is included in the scope of the present invention. Specifically, the peptide may include an amino acid sequence having 80% or more, and more preferably 90% or more sequence homology with the amino acid sequence of SEQ ID NO: 1. GnRH consists of 10 amino acids, and is known to be composed of almost the same amino acids in all animals. GnRH is produced in the hypothalamus and acts on the pituitary gland to promote the production of sex hormones FSH and LH, and when an animal is inoculated using GnRH as an antigen, an antibody recognizing GnRH is formed, and the antibody removes GnRH present in the body, thereby blocking sexual maturation of male and female animals and causing a neutering effect.

The term "recombinant protein" or "fused recombinant protein" used herein refers to a protein in the form of two or more artificially connected proteins, and in the present invention, it refers to a protein in the form in which CTB and GnRH are connected. The fused recombinant protein may be obtained by chemical synthesis after each partner is determined, or expression and purification by a gene recombination method. Preferably, it can be obtained by expressing a fusion gene in which a polynucleotide encoding CTB and a polynucleotide encoding GnRH are connected in a cell expression system.

The GnRH used in the recombinant protein of the present invention preferably has two or more copies rather than one copy of the GnRH amino acid sequence. Since it is possible to further enhance the immunogenicity against GnRH when the GnRH has multiple copies, the amino acid sequence of GnRH, preferably, the amino acid sequence of SEQ ID NO: 1 may take the form of two or more multimers. The number of GnRH copies is not particularly limited as long as it can be accommodated in an expression vector. Preferably, 2 to 20, and more preferably, 2 to 10 copies of GnRH are connected to take the form of a multimer, and even more preferably, 6 copies of GnRH are connected to take the form of a hexamer. In an exemplary embodiment of the present invention, the recombinant protein is manufactured by repeating six GnRH proteins in a row. The polypeptide in which six GnRHs are connected may consist of an amino acid sequence of SEQ ID NO: 2, but the present invention is not limited thereto.

In addition, in such as fusion recombinant protein, CTB and GnRH may be directly connected or connected to each other by an amino acid connector such as a linker. In addition, even when multiple copies of GnRH are included, each GnRH may be directly connected or connected to each other by a connector such as linker. When the GnRH copies are connected by a linker, it is preferable that overall immunogenicity caused thereby is not reduced.

The term "linker" used herein refers to a peptide inserted between proteins such that the activity of each protein can be improved by enhancing the structural flexibility of these proteins when a recombinant protein is formed by connecting CTB and GnRH or when multiple copies of GnRH are connected. As long as the linker can minimize an immune response, the type or number of amino acids is not limited, but preferably 1 to 20, and more preferably 1 to 5 amino acids are used. In an exemplary embodiment of the present invention, in order to connect each protein with each other, the sequence of a restriction enzyme site in the multi-cloning site of a vector, or an alanine-glycine-alanine (AGA) linker was used.

The term "vaccine" used herein is a biological agent containing an antigen that causes an immune response in a living body, and refers to an immunogen that generates immunity in a living body through injection or oral administration in an animal to prevent an infection or induce an antibody against a specific antigen. The animal is a non-human animal, and examples thereof will be listed below.

The term "vaccine composition" used herein may be used after being formulated as an oral formulation such as a powder, a granule, a tablet, a capsule, a suspension, an emulsion, a syrup, or an aerosol, and a sterilized injectable solution according to a conventional method. In the case of formulation, the vaccine composition may be prepared with a diluent or an excipient such as a filler, a thickening agent, a binder, a wetting agent, a disintegrant, a surfactant, which are conventionally used. A solid formulation for oral administration may be a tablet, pill, powder, granule or capsule, and such a solid formulation may be prepared by mixing at least one of excipients, for example, starch, calcium carbonate, sucrose, lactose and gelatin. Also, in addition to the simple excipient, lubricants such as magnesium stearate and talc may also be used. As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration may be a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used.

Administration routes for the vaccine composition according to the present invention may include, but are not limited to, oral, intravenous, intramuscular, intraarterial, intramedullary, intrathecal, intracardiac, transdermal, subcutaneous, intraperitoneal, intranasal, enteral, local, sublingual or rectal administration. Oral or parenteral administration is preferable. The term "parenteral" used herein means subcutaneous, intravenous, intramuscular, intraarticular, intrabursal, intrasternal, intrathecal, intralesional and intracranial injection or infusion techniques. The vaccine composition of the present invention may be administered in the form of a suppository for rectal administration, but it is not limited to the administration methods listed above, and the active material may be administered by any device that can deliver it to a target cell. The vaccine composition according to the present invention may induce a humoral or optionally cell-mediated immune response and/or a combination of the two immune responses.

The dosage of the vaccine composition according to the present invention is selected in consideration of the subject's age, body weight, sex, and physical condition. The amount required to induce an immune response in a subject without any side effects may vary depending on the presence of any recombinant protein used as an immunogen and an excipient.

Meanwhile, the vaccine composition for neutering an animal according to the present invention may consist of an antigen, a pharmaceutically acceptable carrier, a suitable adjuvant, and other conventional substances, and is administered at an immunologically effective amount. The term "immunologically effective amount" used herein refers to an amount sufficient to produce a neutering effect in animals and an amount sufficient not to cause side effects or a serious or excessive immune response, and an exact administration concentration may depend on a specific immunogen to be administered and may be determined by those of ordinary skill in the art using a known method to test the development of an immune response. In addition, the administration concentration may change depending on the dosage form and route, the recipient's age, health and body weight, the characteristics and severity of symptoms, the type of current treatment, and the number of treatments.

In addition, in the recombinant protein according to the present invention, a porcine Fc fragment (pFc2) may be further fused. The pFc2 may be fused in the C-terminus direction of GnRH, but the present invention is not limited thereto.

The term "Fc fragment" used herein refers to a part of an immunoglobulin which has heavy chains (H chains) only connected by an S-S bond and no antigen-binding site when being digested by papain, and the Fc fragment is preferably a porcine Fc fragment, and more preferably, a porcine Fc fragment consisting of the amino acid sequence of SEQ ID NO: 6, but there is no limitation as long as it is an Fc fragment that increases the expression level and solubility of a recombinant protein when being fused with the recombinant protein. In addition, as the Fc fragment of the present invention, a variant of SEQ ID NO: 6 is included in the scope of the present invention. Specifically, the peptide may include an amino acid sequence having 90% or more, more preferably 95% or more, and most preferably 98% or more sequence homology with the amino acid sequence of SEQ ID NO: 6.

In addition, in the recombinant protein according to the present invention, a chaperone binding protein (BiP) may be further fused. The BiP may be fused in the N-terminus direction of GnRH or CTB, but the present invention is not limited thereto.

The term "chaperone binding protein (BiP)" is used to move the expressed recombinant protein to the endoplasmic reticulum, and preferably, BiP consisting of the amino acid sequence of SEQ ID NO: 8, or BiP that moves a recombinant protein to the endoplasmic reticulum when fused with the recombinant protein can be used without limitation. In addition, as the BiP of the present invention, a variant of SEQ ID NO: 8 is included in the scope of the present invention. Specifically, the peptide may include an amino acid sequence having 90% or more, more preferably, 95% or more, and most preferably 98% or more sequence homology with the amino acid sequence of SEQ ID NO: 8. The BiP may be truncated during or after expression so that only a part of the peptide may remain.

In addition, the recombinant protein according to the present invention may further be fused with His-Asp-Glu-Leu (HDEL). The HDEL may be fused in the C-terminus direction of GnRH or pFc2, but the present invention is not limited thereto.

The term "HDEL" used herein refers to a target peptide sequence consisting of histidine-aspartic acid-glutamic acid-leucine, and the HDEL prevents a recombinant protein from being secreted from the endoplasmic reticulum, or allows a recombinant protein to be stably maintained in the endoplasmic reticulum. The HDEL may be encoded by a base sequence of SEQ ID NO: 11, but the present invention is not limited thereto.

In addition, the recombinant protein according to the present invention may be prepared by sequentially fusing BiP, CTB, GnRH, pFc2, and HDEL, and thus the recombinant protein may consist of an amino acid sequence of SEQ ID NO: 12, but the present invention is not limited by the fusion sequence or amino acid sequence.

In addition, the vaccine composition according to the present invention has an excellent effect of neutering a dog or cat.

In addition, the vaccine composition according to the present invention may include one or more adjuvants for improving or reinforcing an immune response.

The term "adjuvant" used herein refers to a material or composition that is added to a vaccine or pharmaceutically active components to increase or affect an immune response. Representatively, the adjuvant usually means a carrier or auxiliary material for an immunogen and/or other pharmaceutically active materials or compositions. Typically, the term "adjuvant" is to be interpreted in a broad sense and refers to a broad range of substances or stratagems capable of enhancing the immunogenicity of an antigen incorporated into or administered the adjuvant. In addition, the adjuvant may be, but is not limited to, classified into an immune potentiator, an antigen delivery system, and a combination thereof. Examples of suitable adjuvants may include aluminum hydroxide, Freud's complete or incomplete adjuvant, DEAE dextran, levamisole, PCG, and poly I:C or poly A:U. In one embodiment of the present invention, as an Emulsigen-based adjuvant, an Emulsigen-D adjuvant including dimethyldioctadecyl ammonium (DDA) bromide was used.

The recombinant protein according to the present invention includes not only a protein having a wild-type amino acid sequence, but also a variant of a fused recombinant protein. The variant of a recombinant protein means a protein having a different sequence from a normal amino acid sequence due to deletion, insertion, non-conservative or conservative substitution of one or more amino acid residues, or a combination thereof. The exchange of an amino acid at a protein or peptide level, which does not overall change the activity of a molecule, is known in the art (H. Neurath et al., 1979). In addition, the recombinant protein may be modified through phosphorylation, methylation, sulfation, acrylation, glycosylation, or farnesylation. The variant or modified form is a functional equivalent that exhibits the same biological activity as a natural protein, but may be a variant or modified form of a protein whose properties are modified, if necessary. Preferably, the variant or modified body is a protein with enhanced structural stability against heat or pH, or increased activity, caused by variation and modification of the amino acid sequence.

The recombinant GnRH protein according to the present invention may be chemically synthesized or produced by a gene recombination method, and preferably, is produced by isolating and purifying a protein expressed by transforming host cells using a recombinant vector.

Therefore, another aspect of the present invention may provide a recombinant vector for neutering an animal, which includes a GnRH gene consisting of a base sequence of SEQ ID NO: 3 and a CTB gene consisting of a base sequence of SEQ ID NO: 5.

The recombinant vector is a carrier into which a foreign DNA fragment is inserted and generally refers to a double-stranded DNA fragment. The "foreign DNA" used herein means DNA originating from a foreign species, or a form substantially modified from an original form when originating from the same species, and a foreign gene is a specific target nucleic acid to be transcribed, which encodes a polypeptide. In order to increase the expression level of a transformed gene in a host cell, in the recombinant vector, the corresponding gene has to be operably linked to transcriptional and translational expression regulatory sequences to exhibit a function in the selected expression host. The recombinant vector is a gene construct including essential regulatory elements operably linked to allow a gene insert to be expressed in cells of a subject, and to manufacture such a gene construct, standard recombinant DNA technology may be used. The type of recombinant vector is not particularly limited as long as it serves to express a target gene and produce a target protein in all types of host cells such as prokaryotic cells and eukaryotic cells, and is preferably a vector that can produce a large amount of a foreign protein having a similar form to the natural state while ensuring a potent promoter and strong expression power. Preferably, the recombinant vector includes at least a promoter, an initiation codon, a gene encoding a target protein, a termination codon, and a terminator. In addition, DNA encoding a signal peptide, an enhancer sequence, untranslated regions (UTRs) at the 5' end and 3' end of the target gene, a selectable marker region, or a replicable unit may be suitably included.

In an exemplary embodiment of the present invention, a recombinant vector manufactured by inserting a CTB gene fragment into a pCAMBIA13000 vector including 6 copies of GnRH gene fragments was transformed into a tobacco plant (*Nicotiana benthamiana*). The vector has a 35S promoter and a heat shock protein (HSP) terminator system, and is a useful vector for mass-producing a protein in a tobacco plant.

The term "vector" used herein refers to a DNA construct containing a DNA sequence operably linked to a suitable regulatory sequence capable of expressing DNA in an appropriate host. The vector may be a plasmid, a phage particle, or simply a potential genome insert. Once transformed into an appropriate host, the vector may replicate and function regardless of a host genome, or in some cases, may be integrated into a genome itself. Since a plasmid is the most commonly used type of vector, the term "plasmid" and "vector" may be sometimes used interchangeably. However, the present invention encompasses other types of vectors that have an equivalent function as known or coming to be known in the art.

In addition, in the present invention, the recombinant vector may further include one or more sequences selected from the group consisting of a polynucleotide encoding pFc2 consisting of an amino acid sequence of SEQ ID NO: 6, a polynucleotide encoding BiP consisting of an amino acid sequence of SEQ ID NO: 8, and a HDEL gene consisting of the base sequence of SEQ ID NO: 11.

Still another aspect of the present invention provides a recombinant protein for neutering an animal prepared using the recombinant vector according to the present invention.

Yet another aspect of the present invention provides a transformant which is transformed with the recombinant vector according to the present invention.

The term "transformation" used herein is a generic term for changes in genetic characteristics of an organism by introduced DNA, and the "transgenic organism or transformant" is an organism produced by injecting an external gene through a molecular genetic method, and preferably, an organism transformed by the recombinant vector of the present invention. The organism is not limited as long as it is used to achieve the purpose of the present invention.

Yet another aspect of the present invention provides a method of neutering an animal, which includes administering the vaccine composition according to the present invention into an animal excluding a human.

The neutering method using the vaccine composition of the present invention can be applied to all animals excluding a human regardless of animal species. The animal is preferably a mammal, and for example, the animal may be a dog, a whale, a cat, a gorilla, a goose, a guinea pig, a pheasant, a camel, a roe deer, a mule, a chicken, a donkey, a dolphin, a pig, a llama, a horse, a buffalo, a deer, a cow, a reindeer, an alpaca, a yak, sheep, a goat, an orangutan, a duck, a monkey, a ferret, a rat, a turkey, an ostrich, or a rabbit, but the present invention is not limited thereto.

In the method of the present invention, administration is preferably performed twice or more. For example, after initial vaccination, booster injections may be performed 1 to 4 times at intervals of 1 to 10 weeks, but this may be suitably modified by those of ordinary skill in the art according to the type of corresponding animal.

Terms and words used in the specification and claims should not be construed as being limited to general or dictionary terms meanings, and should be interpreted with the meaning and concept in accordance with the technical idea of the present invention based on the principle that the inventors have appropriately defined the concepts of terms in order to explain the invention in the best way.

Hereinafter, to help in understanding the present invention, exemplary examples will be suggested. However, the following examples are merely provided to more easily understand the present invention, and not to limit the present invention.

### [Examples]

### Example 1: Expression and purification of BiP-CTB-GnRH-pFc2-HDEL fusion protein

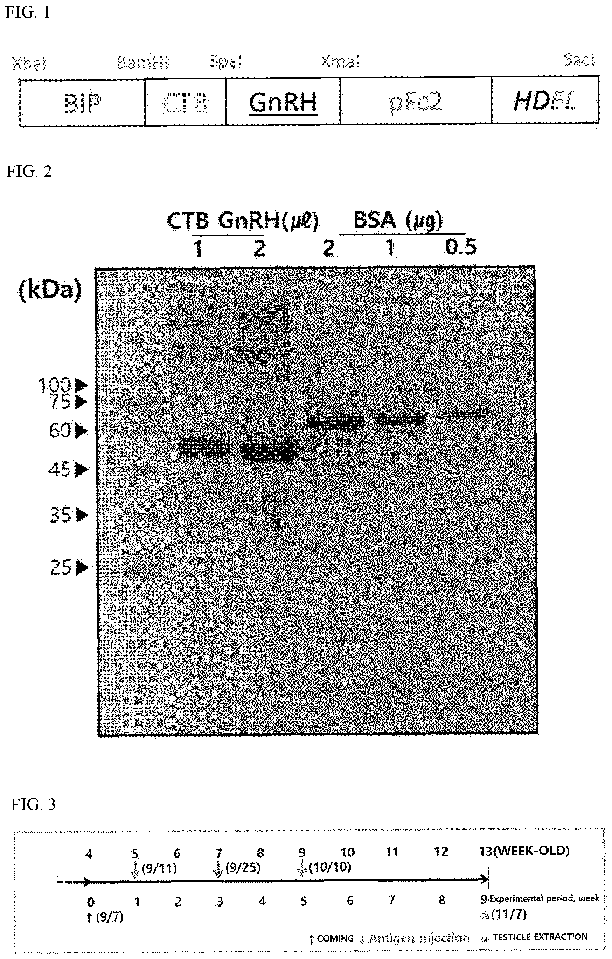

To induce immunogenicity against GnRH, as shown in FIG. 1, a vector for expressing recombinant GnRH in which CTB, pFc2, BiP and HDEL were fused with GnRH was manufactured. Six GnRHs consisting of 10 amino acids each were consecutively connected by alanine-glycine-alanine (AGA) linkers. The amino acid sequence and base sequence of GnRH, which was repeated 6 times, are shown in SEQ ID NO: 2 and SEQ ID NO: 3, respectively. In addition, the amino acid sequence and base sequence of CTB are shown in SEQ ID NO: 4 and SEQ ID NO: 5, respectively, the amino acid sequence and base sequence of pFc2 are shown in SEQ ID NO: 6 and SEQ ID NO: 7, respectively, the amino acid sequence and base sequence of BiP are shown in SEQ ID NO: 8 and SEQ ID NO: 9, respectively, the amino acid sequence and base sequence of HDEL are shown in SEQ ID NO: 10 and SEQ ID NO: 11, respectively, and the total amino acid sequence and base sequence of an expression cassette are shown in SEQ ID NO: 12 and SEQ ID NO: 13, respectively.

Transient expression was induced by inoculating a leaf of tobacco plant with *Agrobacterium tumefaciens* transformed with the above vector. After extracting total protein from the plant leaves and centrifuging, a soluble protein in the solution was isolated using AKTA prime (GE Healthcare, USA) equipped with a protein A-sepharose column, thereby obtaining a pFc2-fused GnRH protein.

### Example 2: Confirmation of concentration of recombinant GnRH protein

To confirm and measure the concentration of the recombinant GnRH protein isolated and purified in Example 1, bovine serum albumin (BSA) whose concentration was known was used as a standard, followed by performing SDS-PAGE. The isolated and purified recombinant GnRH protein was loaded at 1 and 2 µl, and 0.5, 1, and 2 µg of BSA was added for quantification, respectively. A gel was stained by Coomassie blue staining, and a band was finally identified.

As a result, as shown in FIG. 2, it was confirmed that the recombinant GnRH protein is well expressed, isolated and purified, and the concentration of the GnRH recombinant protein is approximately 2 µg/µl. The recombinant GnRH protein identified as described above was used in animal tests of Examples 3 and 4.

### Example 3: Confirmation of effect of recombinant GnRH protein administration - male

### 3.1. Experimental animals, administration conditions and schedule

To confirm the neutering effect of the recombinant GnRH protein, a vaccine composition including the recombinant GnRH protein according to the present invention was administered into a 4-week-old male Sprague Dawley (SD) rat according to a schedule shown in FIG. 3 under administration conditions listed in Table 1 below. As a control, the same amount of a phosphate-buffered saline (PBS) was administered, and an experimental group was administered 100 µg/180 µl of the vaccine composition and 20 µl of Emulsigen^{®}-D adjuvant (see Table 1 below). After acclimation of the provided 4-week-old rat for approximately 4 days, administration was performed a total of three times at intervals of 14 or 15 days. Blood was collected before administration and at the end of the experiment, and 4 weeks after the third administration, testes were extracted to measure weights and sizes, and the concentrations of testosterone in the blood were measured.

**[Table 1]**

| | | Control | Experimental group (recombinant GnRH protein) |
|---|---|---|---|
| Administration | Antigen | 180 µl of PBS | 100 µg/180 µl |
| | Adjuvant | 20 µl | 20 µl |
| Number of experimental subjects | | 3 | 3 |

### 3.2. Visual evaluation of testes

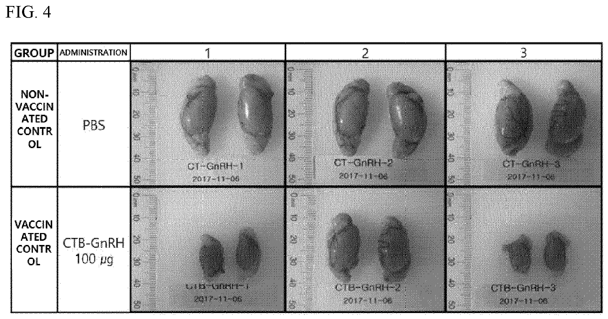

Four weeks after the third administration of the vaccine composition according to the present invention, the testes were extracted to perform visual evaluation. The result is shown in Table 2 below and FIG. 4.

**[Table 2]**

| Group | Administration | No. of subjects | Testes | | Body Weight (g) | Weight ratio | Length ratio |
|---|---|---|---|---|---|---|---|
| | | | Weight (g) | Length (mm) | | (%)^{∗∗} | (%)^{∗∗∗} |
| Nonvaccinated group | PBS | 1 | 2.90 | 37.5 | 461.7 | 0.63 | 103.31 |
| | | 2 | 2.59 | 35.5 | 483.7 | 0.54 | 97.80 |
| | | 3 | 2.52 | 36.0 | 443.2 | 0.57 | 99.17 |
| Vaccinated group | CTB-GnRH 100 µg | 1 | 0.74 | 23.5 | 396.5 | 0.19 | 64.74 |
| | | 2 | 1.98 | 31.5 | 458.1 | 0.43 | 86.78 |
| | | 3 | 0.06 | 22.0 | 383.2 | 0.16 | 60.61 |
| *Weight, length: average of right and left testes (including epididymis) | | | | | | | |
| **Weight ratio (%) = average weight of testes of each subject/total body weight of each subject x 100% | | | | | | | |
| ***Length ratio (%) = average length of testes of each subject/average length of testes of control x 100% | | | | | | | |

Referring to Table 2 and FIG. 4, compared with the non-vaccinated control group, it was confirmed that the weight and length of the testes were significantly reduced in the vaccinated group administered the vaccine composition containing the recombinant GnRH protein. In addition, as can be confirmed from the weight ratio, the ratio of the weight of testes relative to the total body weight of a subject was considerably lower in the vaccinated group, and the average length of testes in the vaccinated group was also 64% to 86% compared to the non-vaccinated group.

The above result confirmed that the animal was successfully neutered by the administration of the vaccine composition including the recombinant GnRH protein according to the present invention.

### 3.3. ELISA result for testosterone

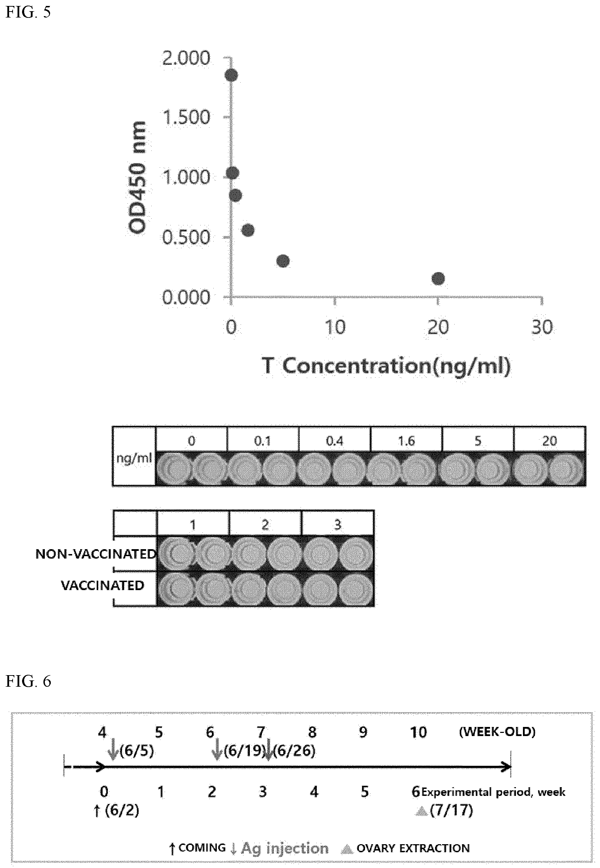

Four weeks after the third administration of the vaccine composition according to the present invention, blood was collected to measure the concentration of testosterone in the blood. The measurement was performed using an ELISA kit (Elabscience) according to the manufacturer's protocol. Specifically, 50 µL of serum was added to a testosterone-coated plate, and the same amount of biotinylated detection antibody was immediately added. The resultant was reacted at 37 °C for 45 minutes, and washed three times using 350 µL of washing buffer. Subsequently, 100 µL of a HRP conjugate solution was added, followed by incubating at 37 °C for 30 minutes. After washing five times in the same manner as described above, 90 µL of a substrate solution was added and incubated at 37 °C for 15 minutes. After that, 50 µL of a reaction stop solution was added, and the OD value was measured at a wavelength of 450 nm through an ELISA machine. In order to quantify the actual amount of testosterone in the blood, the reaction was carried out by including a testosterone standard protein at 0.1, 0.4, 1.6, 5, and 20 ng/ml. A standard graph was made with the OD values obtained as above, and testosterone was quantified by substituting the OD value of the sample obtained above. The result is shown in Table 3 below and FIG. 5.

**[Table 3]**

| Standard (ng/ml) | OD450 nm | | Group | Administration | Subject | OD450 nm |
|---|---|---|---|---|---|---|
| 0 | 1.852 | | Non-vaccinated group | PBS | 1 | 0.171 |
| 0.1 | 1.037 | | | | 2 | 0.150 |
| 0.4 | 0.850 | | | | 3 | 0.194 |
| 1.6 | 0.559 | | Vaccinated group | CTB-GnRH 100 µg | 1 | 0.491 |
| 5 | 0.302 | | | | 2 | 0.540 |
| 20 | 0.154 | | | | 3 | 0.530 |

As a result, as shown in Table 3 and FIG. 5, compared to the non-vaccinated control, the vaccinated group administered the vaccine composition including the recombinant GnRH protein showed a significant decrease in the concentration of testosterone in the blood.

This result is consistent with the result of Example 3.2, and confirmed that neutering is successfully accomplished by the administration of the vaccine composition including the recombinant GnRH protein according to the present invention.

### Example 4: Confirmation of effect of recombinant GnRH protein administration - female

### 4.1. Experimental animals, administration conditions and schedule

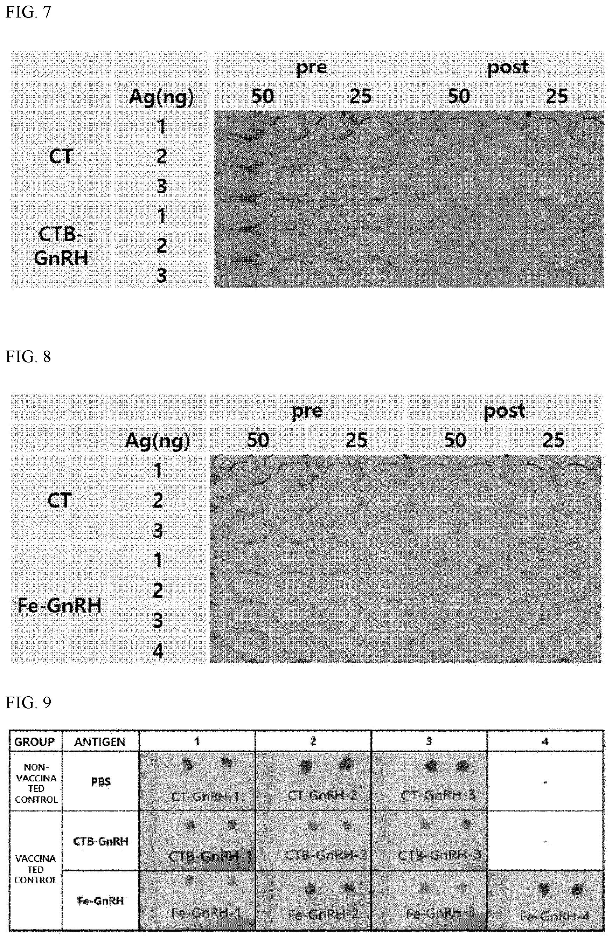

To confirm the neutering effect of the recombinant GnRH protein, a vaccine composition including the recombinant GnRH protein according to the present invention was administered into a 4-week-old male Sprague Dawley (SD) rat according to a schedule shown in FIG. 7 under administration conditions listed in Table 5 below. As a control, the same amount of a phosphate-buffered saline (PBS) was administered, and a comparative group was administered 100 µg/180 µl of a vaccine composition including a protein fused with Fe instead of CTB and 20 µl of Emulsigen^{®}-D adjuvant. An experimental group was administered 100 µg/180 µl of the vaccine composition according to the present invention and 20 µl of Emulsigen^{®}-D adjuvant (see Table 5 below). The vaccine was administered into the 4-week-old rat a total of three times at intervals of 14 days or 7 days. Blood was collected before administration and at the end of the experiment, and three weeks after the third administration, ovaries were extracted to measure weights and sizes, and concentrations of progesterone and estradiol in the blood were measured.

**[Table 4]**

| | | Control | Comparative Group (Fe-GnRH) | Experimental Group (CTB-GnRH) |
|---|---|---|---|---|
| Administration | Antigen | PBS 180 µl | 100 µg/180 µl | 100 µg/180 µl |
| | Adjuvant | 20 µl | 20 µl | 20 µl |
| No. of experimental subjects | | 3 | 4 | 3 |

### 4.2. Confirmation of antibody formation by GnRH

To confirm whether an antibody against GnRH was produced by the neutering vaccine according to the present invention, 96-well plates were coated with GnRH antigens at 50 and 25 ng/well, respectively. As a control, serum obtained from a rat injected with the same amount of PBS was used.

As a primary antibody, serum of a CTB-GnRH or Fe-GnRH-administered rat was diluted at a ratio of 1:100, followed by reaction at 37 °C for 2 hours. Afterward, an anti-rat secondary antibody (1:5000) was treated, and then the chromogenic substance, 3,3',5,5'-tetramethylbenzidine (TMB) was treated.

As a result, as shown in Tables 5 and 6 and FIGS. 7 and 8, both CTB-GnRH and Fe-GnRH showed reactivity, confirming that the antibody against GnRH was successfully generated.

**[Table 5]**

| | | **Before administration** | | **After administration** | |
|---|---|---|---|---|---|
| | **Antigen(ng)** | **50ng** | **25ng** | **50ng** | **25ng** |
| **Control** | **1** | 1.091 | 0.672 | 1.728 | 1.216 |
| | **2** | 2.929 | 2.064 | 2.081 | 1.766 |
| | **3** | 0.487 | 0.279 | 1.888 | 1.161 |
| **CTB-GnRH** | **1** | 2.564 | 1.543 | 4.313 | 4.172 |
| | **2** | 2.574 | 1.672 | 4.451 | 4.285 |
| | **3** | 0.300 | 0.169 | 4.373 | 4.365 |

**[Table 6]**

| | | **Before administration** | | **After administration** | |
|---|---|---|---|---|---|
| | **Antigen(ng)** | **50ng** | **25ng** | **50ng** | **25ng** |
| **Control** | **1** | 0.369 | 0.217 | 0.691 | 0.457 |
| | **2** | 1.392 | 0.843 | 0.742 | 0.490 |
| | **3** | 0.191 | 0.148 | 0.790 | 0.443 |
| **Fe-GnRH** | **1** | 0.555 | 0.438 | 4.611 | 4.209 |
| | **2** | 0.552 | 0.437 | 4.290 | 4.246 |
| | **3** | 0.240 | 0.153 | 4.302 | 4.080 |
| | **4** | 0.256 | 0.146 | 3.087 | 2.278 |

### 4.3. Visual evaluation of ovaries

Three weeks after the third administration of the vaccine composition according to the present invention, ovaries were extracted to perform visual observation. The result is shown in Table 7 below and FIG. 9.

**[Table 7]**

| Group | Administration | No. of subjects | Ovaries | | Body Weight (g) | Weight ratio | Length ratio |
|---|---|---|---|---|---|---|---|
| | | | Length (mm) | Weight (g) | | (%)^{∗∗} | (%)^{∗∗∗} |
| Nonvaccinated group | PBS | 1 | 0.68 | 0.0674 | 251.4 | 0.027 | 100.75% |
| | | 2 | 0.68 | 0.0918 | 250.8 | 0.037 | 100.75% |
| | | 3 | 0.65 | 0.0654 | 248.8 | 0.026 | 97.01% |
| Vaccinated group | CTB-GnRH 100 µg | 1 | 0.53 | 0.0318 | 258.5 | 0.012 | 78.36% |
| | | 2 | 0.53 | 0.0350 | 268.9 | 0.013 | 78.36% |
| | | 3 | 0.45 | 0.0377 | 244.9 | 0.015 | 67.16% |
| | Fe-GnRH 100 µg | 1 | 0.48 | 0.0283 | 310.7 | 0.009 | 70.90% |
| | | 2 | 0.60 | 0.0465 | 253.7 | 0.018 | 89.55% |
| | | 3 | 0.58 | 0.0484 | 235.9 | 0.021 | 85.82% |
| | | 4 | 0.58 | 0.0645 | 225.9 | 0.029 | 85.82% |
| *Weight, length: average of right and left ovaries | | | | | | | |
| **Weight ratio (%) = average weight of ovaries of each subject/total body weight of each subject x 100% | | | | | | | |
| ***Length ratio (%) = average length of ovaries of each subject/average length of ovaries of control x 100% | | | | | | | |

As shown in Table 7 and FIG. 9, compared to the non-vaccinated control, it was confirmed that, in the vaccinated group administered the vaccine composition including the recombinant GnRH protein, the weight and length of the ovaries were significantly reduced. That is, as can be confirmed from the weight ratio, the ratio of the weight of the ovaries relative to the total body weight of the subject was significantly lower in both vaccinated groups, and the length of the ovaries was also 67% to 89% as compared to the non-vaccinated group.

Particularly, compared to Fe-GnRH, CTB-GnRH showed a better ovarian atrophy effect. When Fe-GnRH was administered, the length ratio in the vaccinated group was 83% on average, whereas when CTB-GnRH was administered, the length ratio in the vaccinated group was 74.6% on average. When the Fe-GnRH was administered, the weight ratio was 0.019% on average, whereas when CTB-GnRH was administered, the weight ratio was 0.013% on average.

The above results confirmed that neutering was successfully performed by administration of the vaccine composition including the recombinant CTB-GnRH protein according to the present invention and that the effect was more excellent when CTB, rather than Fe, was fused.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

The present invention relates to a vaccine composition for neutering an animal, which includes a recombinant protein in which a cholera toxin B subunit (CTB) and gonadotropin-releasing hormone (GnRH) are fused, and an antibody against GnRH was induced into a subject administered the recombinant protein including the polypeptide sequence of GnRH, thereby confirming ovarian and testicular atrophy. Therefore, the present invention is expected to have great industrial applicability because it can be effectively used for neutering an animal at low cost and with high safety and minimal side effects.

## Claims

1. A vaccine composition for neutering an animal, comprising:
a recombinant protein in which a cholera toxin B subunit (CTB) and n copies of gonadotropin-releasing hormone (GnRH) are fused as an active ingredient,
wherein n is an integer from 1 to 20.

2. The vaccine composition of claim 1, wherein the GnRH consists of an amino acid sequence of SEQ ID NO: 1.

3. The vaccine composition of claim 1, wherein the CTB consists of an amino acid sequence of SEQ ID NO: 4.

4. The vaccine composition of claim 1, wherein n is 6.

5. The vaccine composition of claim 4, wherein the GnRH consists of an amino acid sequence of SEQ ID NO: 2.

6. The vaccine composition of claim 1, wherein, when n is 2 or more,
the GnRH is connected with each other by alanine-glycine-alanine linkers.

7. The vaccine composition of claim 1, wherein in the recombinant protein, a porcine Fc fragment (pFc2) is further fused.

8. The vaccine composition of claim 7, wherein the pFc2 consists of an amino acid sequence of SEQ ID NO: 6.

9. The vaccine composition of claim 8, wherein the CTB is fused in the N-terminus direction of GnRH, and the pFc2 is fused in the C-terminus direction of GnRH.

10. The vaccine composition of claim 1, wherein in the recombinant protein, a chaperone binding protein (BiP) is further fused.

11. The vaccine composition of claim 10, wherein the BiP consists of an amino acid sequence of SEQ ID NO: 8.

12. The vaccine composition of claim 10, wherein the CTB is fused in the N-terminus direction of GnRH, and the BiP is fused in the N-terminus direction of CTB.

13. The vaccine composition of claim 1, wherein in the recombinant protein, His-Asp-Glu-Leu (HDEL) is further fused.

14. The vaccine composition of claim 13, wherein the HDEL is encoded by a base sequence of SEQ ID NO: 11.

15. The vaccine composition of claim 13, wherein the CTB is fused in the N-terminus direction of GnRH, and the HDEL is fused in the C-terminus direction of GnRH.

16. The vaccine composition of claim 1, wherein in the recombinant protein, BiP, CTB, GnRH, pFc2, and HDEL are sequentially fused.

17. The vaccine composition of claim 16, wherein the recombinant protein consists of an amino acid sequence of SEQ ID NO: 12.

18. The vaccine composition of any one of claims 1 to 17, further comprising an adjuvant.

19. The vaccine composition of claim 18, wherein the adjuvant is an Emulsigen-based adjuvant.

20. A method of neutering an animal, comprising:
administering the vaccine composition of any one of claims 1 to 17 into an animal excluding a human.

21. The method of claim 20, wherein the animal is a dog, a whale, a cat, a gorilla, a goose, a guinea pig, a pheasant, a camel, a roe deer, a mule, a chicken, a donkey, a dolphin, a pig, a llama, a horse, a buffalo, a deer, a cow, a reindeer, an alpaca, a yak, sheep, a goat, an orangutan, a duck, a monkey, a ferret, a rat, a turkey, an ostrich, or a rabbit.

22. A recombinant vector for neutering an animal, comprising:
a GnRH gene consisting of a base sequence of SEQ ID NO: 3 and a CTB gene consisting of a base sequence of SEQ ID NO: 5.

23. The recombinant vector of claim 22, further comprising:
a polynucleotide encoding pFc2 consisting of an amino acid sequence of SEQ ID NO: 6.

24. The recombinant vector of claim 22 or 23, further comprising:
a polynucleotide encoding BiP consisting of an amino acid sequence of SEQ ID NO: 8.

25. The recombinant vector of claim 24, further comprising:
a HDEL gene consisting of a base sequence of SEQ ID NO: 11.

26. The recombinant vector of claim 25, comprising:
a polynucleotide encoding an amino acid sequence of SEQ ID NO: 12, or a base sequence of SEQ ID NO: 13.

27. A recombinant protein for neutering an animal prepared using the recombinant vector of claim 22.

28. A transformant transformed with the recombinant vector of claim 22.

29. A neutering method comprising:
administering a vaccine composition comprising a recombinant protein in which a cholera toxin B subunit (CTB) and n copies of gonadotropin-releasing hormone (GnRH) are fused as an active ingredient into a subject excluding a human,
wherein n is an integer from 1 to 20.

30. A use of a recombinant protein in which a cholera toxin B subunit (CTB) and n copies of gonadotropin-releasing hormone (GnRH) are fused for animal neutering,
wherein n is an integer from 1 to 20.

31. A use of a recombinant protein in which a cholera toxin B subunit (CTB) and n copies of gonadotropin-releasing hormone (GnRH) are fused for the manufacture of a vaccine used in animal neutering,
wherein n is an integer from 1 to 20.
